(19) Europäisches Patentamt
European Patent Office
Office européen des brevets

(11) Publication number: **0 358 129 B1**

(12) # EUROPEAN PATENT SPECIFICATION

(45) Date of publication of patent specification: **30.12.92**

(51) Int. Cl.5: **C07D 323/00**, B01J 20/22, C07B 57/00

(21) Application number: **89116202.6**

(22) Date of filing: **01.09.89**

(54) **New Crown ether compound and separating agent.**

(30) Priority: **05.09.88 JP 221879/88**

(43) Date of publication of application:
**14.03.90 Bulletin 90/11**

(45) Publication of the grant of the patent:
**30.12.92 Bulletin 92/53**

(84) Designated Contracting States:
**CH DE LI**

(56) References cited:
**DE-A- 3 707 291**
**GB-A- 1 452 182**

**PATENT ABSTRACTS OF JAPAN, vol. 10, no. 28 (C-326)(2085), 4 February 1986; & JP-A-60 181 034 (DAICEL KAGAKU KOGYO K.K.) 14.09.1985**

**ANGEWANDTE CHEMIE, vol. 100, no. 12, 1988, pages 1775-1777; F. DIEDERICH et al.: "Auf 2,2',7,7'-Tetrahydroxy-1, 1'-binaphthyl basierende neuartige chirale mono- und ditope cyclophanartige Wirtverbindungen mit einer unpolaren Bindungsstelle"**

(73) Proprietor: **Director General, Agency of Industrial Science and Technology**
**1-3-1, Kasumigaseki Chiyoda-ku**
**Tokyo 100(JP)**

Proprietor: **DAICEL CHEMICAL INDUSTRIES, LTD.**
**No. 1-Banchi, Teppo-cho**
**Sakai-shi Osaka-fu 590(JP)**

(72) Inventor: **Shinbo, Toshio Nat. Chem. Lab. for Ind.**
**Agency Ind. Science and Techn. 1, Higashi 1-chome**
**Tsukuba-shi Ibaraki(JP)**
Inventor: **Yamaguchi, Tomohiko Nat. Chem. Lab. for Ind.**
**Mendestr. 8**
**4600 Dortmund 1(DE)**
Inventor: **Tachibana, Kouzou**
**940, Shinzaike Aboshi-ku**
**Himeji-shi Hyogo(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 358 129 B1

JOURNAL OF THE AMERICAN CHEMICAL SOCIETY, vol. 99, no. 8, 1304.04.1977, pages 2564-2571; E.P. KYBA et al.: "Host-Guest Complexation. 1.Concept and Illustration"

(74) Representative: **Patentanwälte Grünecker, Kinkeldey, Stockmair & Partner**
**Maximilianstrasse 58**
**W-8000 München 22(DE)**

## Description

Industrial Utilization Field

This invention relates to a new crown ether compound. In addition, this invention relates to a separating agent in which said compound is adsorbed onto a carrier, and particularly to a filler for separation of optical isomers.

Crown ether compounds that do not possess substituents $R_2$ and $R_3$ in general formula (I) indicated in the claim of the invention are commonly known. In addition, filling agents for separation of optical isomers that use these are also commonly known (refer to Pat. Disclosure DE-A-3707291).

However, since the adsorption of separating agents which use commonly known crown ether compounds coated one a carrier is weak, these separating agents have the problem of poor durability.

The inventors developed a new crown ether compound in order to solve the related problems and provide a separating agent having superior durability which uses this compound.

In other words, this invention relates to a crown ether compound represented by the general formula (I) indicated below,

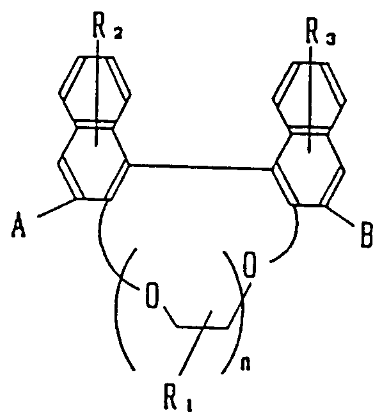

( I )

wherein A and B which may be the same or different represent hydrogen atom, a straight chain or branched alkyl group having 1-6 carbon atoms, an aryl group having 6-10 carbon atoms, or an aralkyl group having 7-9 carbon atoms;

$R_1$ represents a hydrogen atom, a straight chain or branched alkyl group having 1-20 carbon atoms, and may be bonded to any carbon atom of the cyclic polyether the carbon number of which is 1-12; n represents an integer from 3-10,

$R_2$ and $R_3$ which may be the same or different represent a straight chain or branched alkyl group having 1-30 carbon atoms, an aryl group having 6-18 carbon atoms, or an aralkyl group having 7-30 carbon atoms, the location of $R_2$ and $R_3$ being anywhere else than the A and B substitution positions on the condensed ring and although one of each $R_2$ and $R_3$ is satisfactory, a maximum of 5 groups may be substituted.

In general formula (I), A and B represent hydrogen atoms, straight chain or branched alkyl groups having 1-6 carbon atoms such as methyl groups, ethyl groups or isopropyl groups, aryl groups having 6-10 carbon atoms such as phenyl groups, or aralkyl groups having 7-9 carbon atoms such as benzyl groups.

In addition, in general formula (I), $R_2$ and $R_3$ preferably represent alkyl groups having 4-30 carbon atoms such as long-chain alkyl groups, for example, $C_8$, $C_{12}$, $C_{16}$ or $C_{18}$.

In addition, examples of aryl groups and aralkyl groups include the same examples as those listed for A and B.

Although $R_2$ and $R_3$ are preferably substituted at the 6,6' position, up to 2-5 groups may also be substituted at other positions.

In other words, the preferable crown ether compound of this invention is indicated with general formula (II) below,

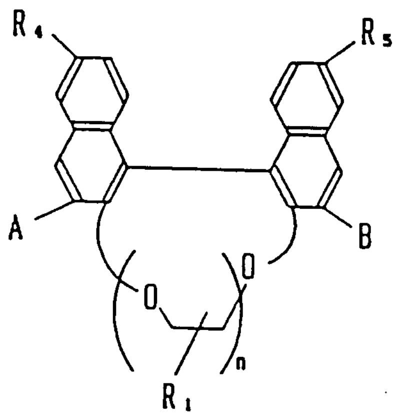

(II)

wherein A, B and $R_1$ are as defined in general formula (I) and $R_4$ and $R_5$ each represent a straight-chain or branched alkyl group having 4-30 carbon atoms.

The crown ether compound of this invention represented by the general formulae (I) and (II) may be either racemic or optically active.

In addition, this invention provides a new separating agent. In other words, the separating agent of this invention relates to a separating agent in which an optically active crown ether compound represented by general formulae (I) and (II) is adsorbed onto a carrier.

Synthesis Method :

The following scheme is an example of the synthesis method of the new crown ether compound of this invention.

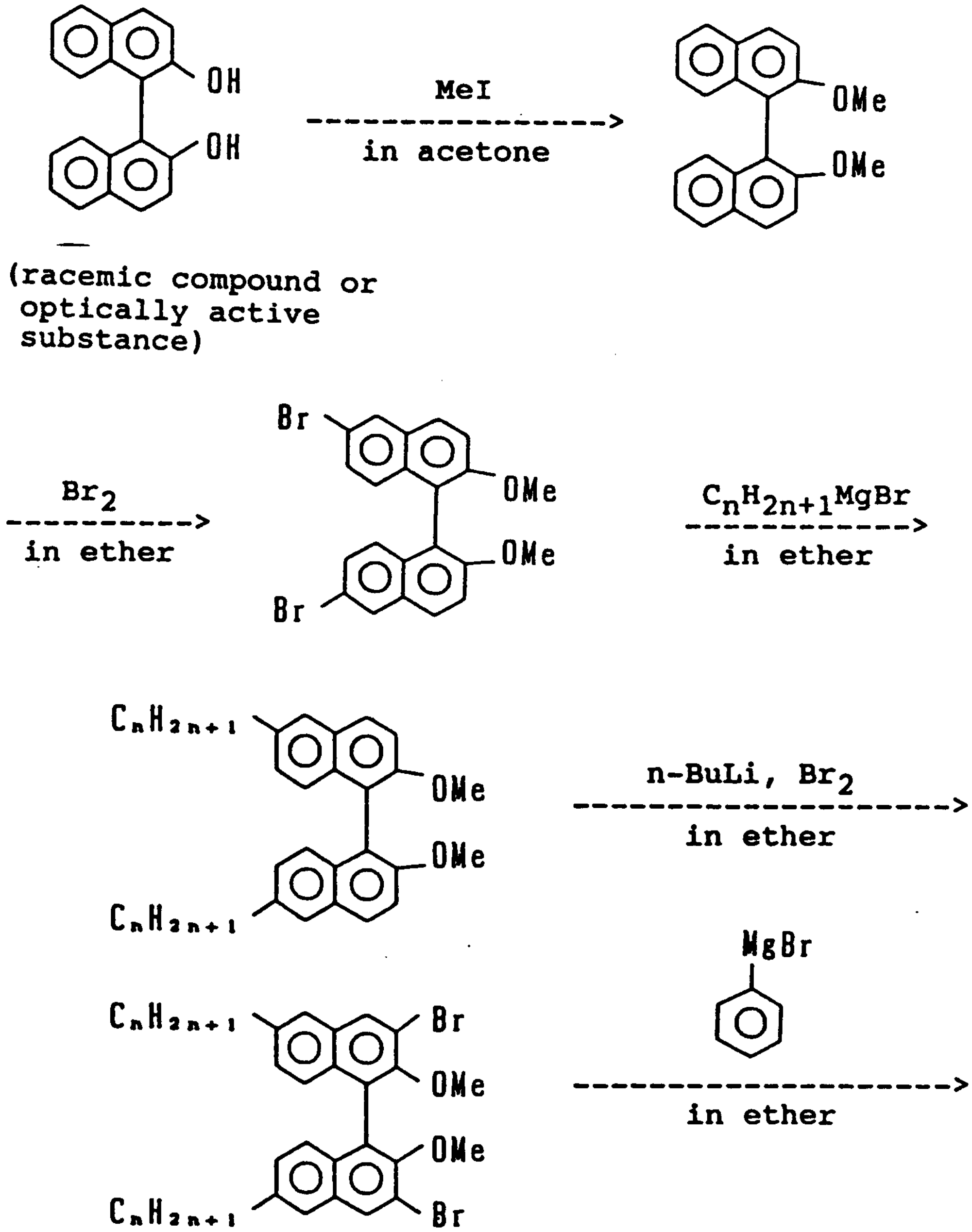
MeI
in acetone
(racemic compound or optically active substance)
Br2
in ether
CnH2n+1MgBr
in ether
n-BuLi, Br2
in ether
in ether

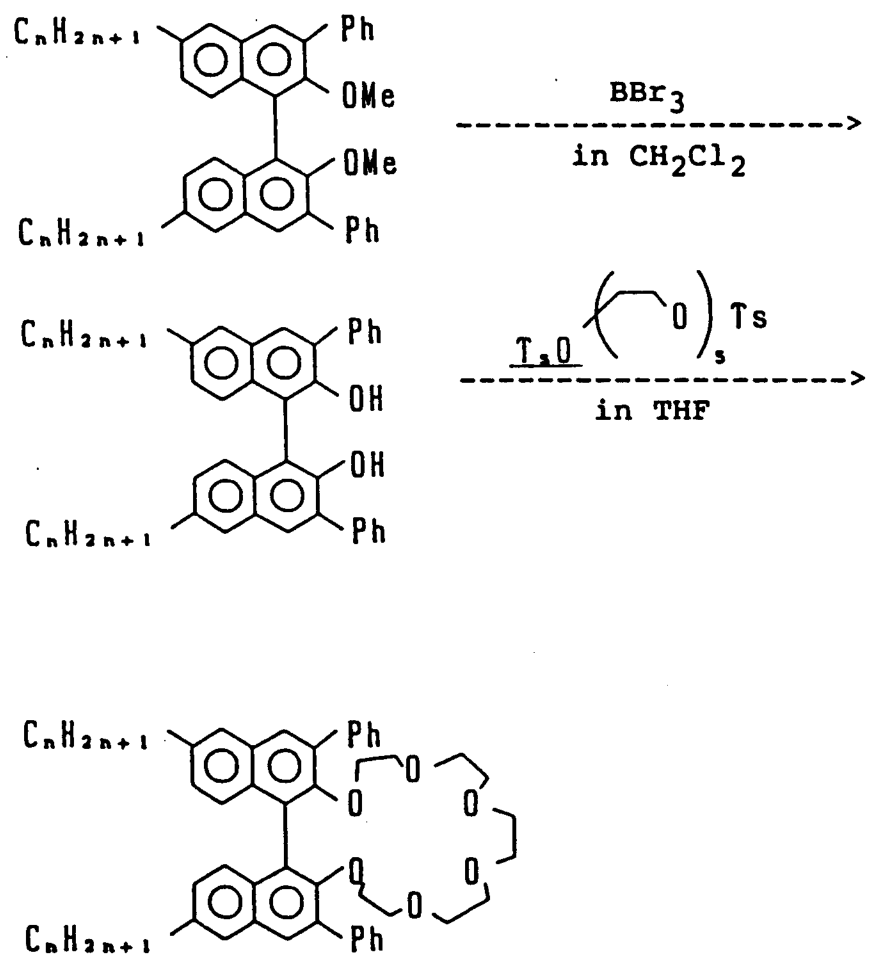


The racemic or optically active crown ether compound in this invention is synthesized, for example, by the procedure indicated below.

First, racemic or optically active binaphthol 1 is used as the starting material. Dialkoxy substance 2 is obtained by reacting this with an excess amount of haloalkyl in an organic solvent such as acetone or tetrahydrofuran in an inert gas atmosphere. Next, by allowing an excess amount of halogen to act on 2 in an organic solvent such as ether or tetrahydrofuran in an inert gas atmosphere, 6,6' dihalo substance 3 is obtained. When 3 is reacted with a suitable Grignard reagent in an organic solvent like tetrahydrofuran or ether in an inert gas atmosphere, 6,6' di-substituted substance 4 is obtained. Further, after allowing butyl lithium and tetramethylethylenediamine to act on 4 in an organic solvent like tetrahydrofuran or ether, then reacted with halogen at a low temperature, 3,3' dihalo substance 5 is obtained. When 5 is reacted with a suitable Grignard reagent in an organic solvent like tetrahydrofuran or ether in an inert gas atmosphere, 3,3' di-substituted substance 6 is obtained. By allowing boron tribromide to act on 6 in an organic solvent like methylene chloride or chloroform, 2,2' dihydroxy substance 7 is obtained. By reacting an equimolar amount of penta- or hexaethylene glycol derivative with 7 in an organic solvent like tetrahydrofuran or ether in an inert gas atmosphere, the target compound, crown ether 8 is obtained.

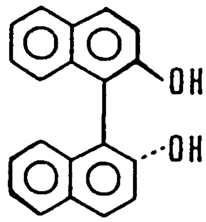

1

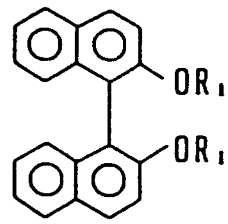

2

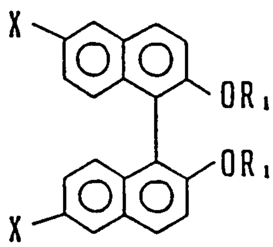

3

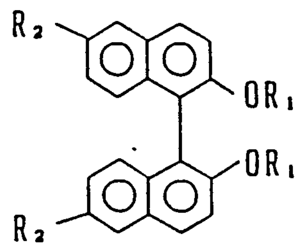

4

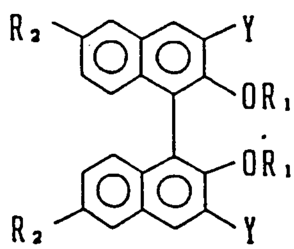

5

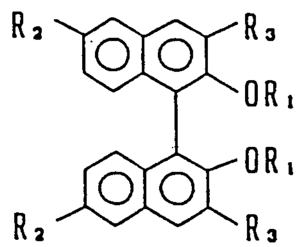

6

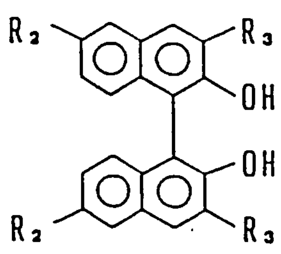

7

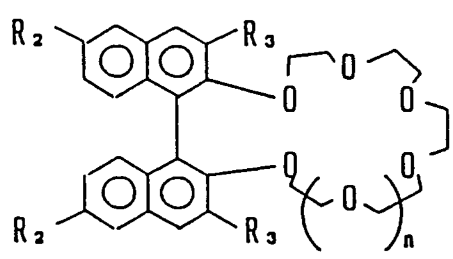

(n = 1 or 2)

8

(Synthesis Method of Separating Agent)

The optically active crown ether compound used in this invention may be either the R or S optical isomers of the compound of general formula (I) and (II) indicated previously.

The carrier onto which the above optically active crown compound of this invention is adsorbed and supported on is either a porous organic carrier or porous inorganic carrier. A carrier having a particle

diameter of 1-1000μm, and preferably 1-300μm, is used. In addition, a porous carrier having an average pore diameter of 10Å-10μm, preferably 50-1000Å, is preferred. Examples of suitable porous organic carriers include polymer substances such as polystyrene, polyacrylamide and polyacrylate. Examples of suitable porous inorganic carriers include synthetic or natural substances such as silica, alumina, magnesia, titanium oxide, glass, silicate and kaolin.

In addition, in this invention, it is preferable that the above lipophilic optically active crown ether is adsorbed on and carried on these carriers treated with a silane treatment agent such as octadecylsilane and diphenyldichlorosilane, or with a silane coupling agent such as phenyldimethoxysilane. In order to obtain satisfactory separation results, the amount of crown compound that is adsorbed on the treated carrier surface should be such that $10^{-6}$ to 0.1 moles or less of crown compound are adsorbed per 1cc of carrier, preferably $10^{-5}$ to $10^{-3}$ moles per 1cc of carrier, but the adsorbing process is not limited thereto.

In order to perform this adsorption and support satisfactorily, the above surface treated carrier is filled into a column and a solution in which the above optical active lipophilic crown compound is dissolved in a mixed solvent, consisting of an organic solvent and water having fixed components, is circulated inside this filled column using a pump. In this case, since the concentration of the crown compound in the circulating solution will decrease with time due to adsorption of the crown compound onto the carrier, after a fixed period of time, more water is added to decrease the solubility of the crown compound in the circulating solution followed by repeated circulation in the column. By sequentially repeating this type of procedure, a filler in which the crown compound is adsorbed at a specified concentration can be directly prepared inside the column. Furthermore, the solvent used as the above organic solvent is one that is compatible with water and dissolves the crown compound. Examples of such organic solvent include alcohols such as methanol, ethanol and propanol, as well as other types of solvents such as acetonitrile and tetrahydrofuran.

In addition to the method indicated above, this adsorption can also be performed using a method in which said crown ether is dissolved in a a solvent which is able to dissolve it, thoroughly mixing this with the carrier, and then removing the solvent by reduced pressure or pressurized air flow, or using a method in which said crown ether is dissolved in a solvent which is able to dissolve it, thoroughly mixing this with the carrier, and dispersing said solvent by stirring and dispersing in a solution that is not compatible with said solvent.

A column filled with a filler of this invention is used for the separation of various kinds of racemic compounds. For example, amino compounds, especially amino compounds pocessing asymmetric centers at the [a] and/or [b] positions of the amino group, can be separated excellently. Specific examples of such compounds include phenylglycine, methionine, leucine, glutamate, phenylalanine, cysteine, tyrosine, alanine and phenylethylamine. In addition, columns containing the filler of this invention can also be used for the separation of other optically active organic ions due to ionic interaction.

Although demineralized water, or dilute aqueous solutions of salt or acid can be used as eluates for the column containing the filler of this invention, dilute acids are preferred since then, in particular, demonstrate considerable separation effects.

Embodiment 1

A: Synthesis of 6,6'-dioctyl-2,2'-dimethoxy-1,1'-binaphthyl

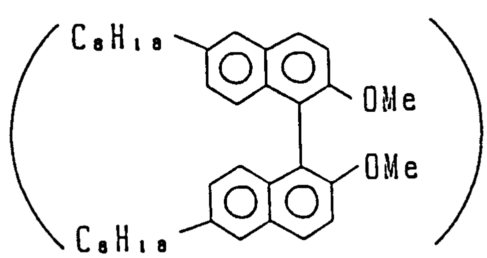

4ml of bromine were dropped to a solution (100ml) of dichloromethane containing 10g of 2,2'-dimethoxy-1,1'-binaphthyl (R isomer) over a period of 20 minutes in a nitrogen atmosphere at -75 °C and were allowed to react for 2.5 hours. Following stirring for 30 minutes at a temperature of 25°C, 100ml of a 10% $Na_2SO_3$ solution were added to decompose the unreacted bromine. 14g of 6,6'-dibromo-2,2'-dimethoxy-1,1'-binaphthyl (Compound 1) were obtained from the isolated cake-form product and the dried

residue resulting from evaporation of the dichloromethane phase (yield: 93%).

63mmol (100ml of ether solution) of $C_8H_{17}MgBr$ were added to a ether solution (150ml) containing 10g of Compounds 1 and 1.25g of dichloro[1,3-bis(diphenylphosphine)propane]nickel in a nitrogen atmosphere, and refluxed for 20 hours. After cooling, 800ml each of dichloromethane and 1M hydrochloric acid were added to obtain an oily reaction product by extraction. By purifying the above reaction product with column chromatography (silica gel, petroleum ether/ethyl ether), 8.1g of pure 6,6'-dioctyl-2.2'-dimethoxy-1,1'-binaphthyl (Compound 2) were obtained (yield: 72%).

B: Synthesis of 6,6'-dioctyl-3,3'-diphenyl-2,2'-dihydroxy-1,1'-binaphthyl

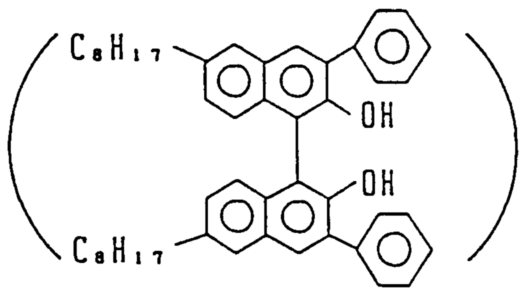

32ml of 1.6M butyl lithium (hexane solution) were added to 400ml of an ether solution containing 5.2g of tetramethylethyleneamine in a nitrogen atmosphere. After allowing the mixture to react for 15 minutes at 25°C, an ether suspension containing 6.8g of Compound 2 was added and the mixture was stirred for 3 hours. After cooling to -78°C, 10ml of bromine dissolved in 30ml of pentane were added over a 10 minutes period. After the reaction liquid had reached room temperature (25°C) and stirred for 1 hour, 300ml of a saturated solution of $Na_2SO_3$ were added and allowed to react for 4 hours. The dichloroethane extract of the mixture was purified with column chromatography (silica gel, cyclohexane/benzene), to give 4.8g of 6,6'-dioctyl-3,3' -dibromo-2,2'-dimethoxy-1,1'-binaphthyl (Compound 3) (yield: 55%).

20mmol of phenylmagnesium bromide (PhMgBr) (30ml of an ether solution) were added to 50ml of ether in which 4.4g of Compound 3 and 0.5g of $Ni[P(C_6H_5)_3]_2Cl_2$ were suspended in a nitrogen atmosphere, and refluxed for 20 hours. After cooling, 500ml each of dichloromethane and 1M hydrochloric acid were added and the layers were separated. The organic layer was dried and evaporated under reduced pressure, and the residue was purified by column chromatography (silica gel, cyclohexane/benzene) to obtain 1.66g of 6,6'-dioctyl-3,3,'- diphenyl-2,2'-dimethoxy-1,1'-diphenyl (Compound 4) (yield: 38%).

1.5g of Compound 4 were then dissolved in 150ml of dichloromethane and after cooling to 0°C, 7.5g of $BBr_3$ were added. This was then allowed to react for 24 hours at 25°C. The reactants were then again cooled to 0°C and after decomposing the excess $BBr_3$ with water, 6,6'-dioctyl-3,3'-diphenyl-2,2'-dihydroxy-1,1'-binaphthyl (Compound 5) was obtained from the organic layer. This was purified by column chromatography to obtain 1.55g (yield: 80%).

C: Synthesis of 2,3:4,5-bis[1,2-(3-phenyl-6-octylnaphtho)]-1,6,9,12,15,18-hexaoxacycloeicosa-2,4-diene

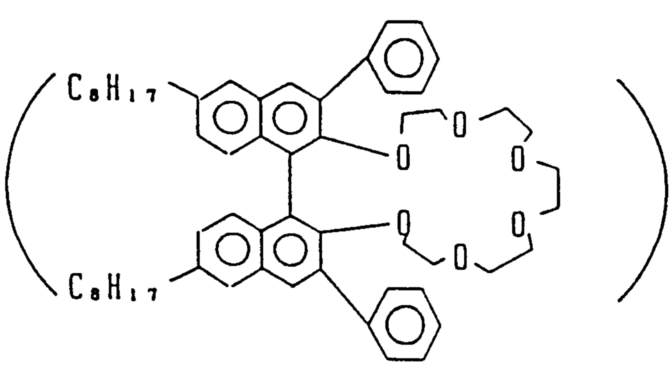

0.24g of KOH were added to 100ml of a tetrahydrofuran solution containing 0.74g of Compound 5 and 0.68g of pentaethyleneglycol ditosylate in a nitrogen atmosphere at 25°C, and refluxed for 72 hours. The reactants were extracted with dichloromethane, evaporated under reduced pressure, and purified with column chromatography (silica gel, petroleum ether/ethyl ether) and gel chromatography (polystyrene/chloroform) to obtain 0.44g of the desired compound, 2,3:4,5-bis[1,2-(3-phenyl-6-octylnaphtho)-]-1,6,9,12,15,18-hexaoxacycloeicosa-2,4-diene (Compound 6) (yield: 46%). The data regarding the physical properties of this Compound 6 are listed below.

* Specific Rotation:

$$[a]^{25}_{589} = +7.7^{\circ}$$

* Mass Spectrum: m/e 864($M^{+}$)

* $^{1}H$ NMR (400MHz, $CDCl_3$)

= 0.87(t,$CH_3$,6H), 1.29(m,other $CH_2$),20H), 1.70(m, $\beta$-$CH_2$,4H), 3,41(m,$OCH_2$,20H), 7.47(m,ArH,18H)

Separation Examples :

In the tables, $k_D'$, $k_L'$, a and $R_s$ are defined as indicated below.

Capacity Factor ($k_D'$, $k_L'$)

$$= \frac{\text{(Retention Volume of each enantiomer)} - \text{(Dead Volume)}}{\text{(Dead Volume)}}$$

separation factor ([a])

$$= \frac{\text{Capacity Factor of the later Eluted Enantiomer}}{\text{Capacity Factor of the Earlier Eluted Enantiomer}}$$

Resolution (Rs)

$$= \frac{2 \times \text{(Distance between Peaks of the Earlier Eluted Enantiomer and the Later One)}}{\text{Total of Bandwidths of Both Peaks}}$$

Separation Example 1

A: Preparation of optical Isomer Separation Column

80mg of the optically active R isomer of the crown compound obtained in Embodiment 1 were dissolved in a 95% aqueous methanol solution (60ml). This solution was then circulated in a commercially available octadecyl silica column (ODS column, Lichrosorb rp-18, diameter: 4mm, length: 125mm, particle diameter: 5μm) for 12 hours using a pump. Water was sequentially added to the circulating liquid to gradually reduce the methanol ratio (the final methanol content of the circulating liquid was approx. 70%). As a result, the crown compound was nearly completely adsorbed onto the ODS in the column.

B: Separation of Optical Isomers

The above optical isomer separation column was connected to a HPLC (Nihon Bunko, Model DIP-1 pump, Soma Optical, Model S-3101A UV Detector, Rheodyne sample injector). Examples of separation of

racemic amino acids and amines are listed in Table 1.

In this case, $10^{-2}$M $HClO_4$ was used as eluate and the column temperatures were 18°C and 2°C. In addition, the sample (amino compound) amount was $10^{-8}$ moles. Further, the amino compound was detected using UV absorption at 200nm or 254nm.

As is shown in Table 1, the L isomer of all the amino acids, was eluted before the D isomer. In contrast, in the case of phenylethylamine, the D isomer was eluted prior to the L isomer. In addition, separation was improved as the column temperature was lowered. All of the more than 20 amino acids that were investigated, were optically separated, with the exception of proline. The separation factor depended upon the concentration of the acid in the eluate. The separation factor increased with an increase in the concentrations of the acid.

Separation Example 2

174mg of the optically active R isomer of the crown compound obtained in Embodiment 1 were dissolved in a 97% aqueous methanol solution (60ml). This solution was then circulated in a commercially available octadecyl silica column (ODS column, Lichrosorb rp-18, diameter: 4mm, length: 125mm, particle diameter: 5um) for 12 hours using a pump. Water was sequentially added to the circulating liquid to gradually reduce the methanol ratio (the final methanol content of the circulating liquid was approx. 70%). As a result, the crown compound was nearly completely adsorbed onto the ODS in the column.

This column was attached to the HPLC described in Separation Example 1 and an optical separation was performed on the amino acids. The results are listed in Table 2 (columns R-2). Table 2 also shows the results using the crown compound (R-1) described in Pat. Disclosure SHO 62-210053 as a comparative example.

The measurement conditions comprised a column temperature of 18ºC $10^{-2}$M $HClO_4$ as eluate and the sample in an amount of $10^{-8}$ moles.

When a crown compound was used having the same number of moles, as can be seen from the table, the column in accordance with this invention (R-2) demonstrated separation capabilities comparable with those of the column of the comparative example (R-1).

The results of an examination of the organic solvent resistance of this column are listed in Table 3. This column demonstrated organic solvent resistance considerably superior to that of the R-1 column. Even when 40% methanol was used as the eluate, elution of the adsorbed crown compound was not detected at all.

Table 4 indicates the results of optical separation of amino acids when a mixed solution of methanol and water (containing $10^{-2}$M $HClO_4$) was used as the eluate.

As the methanol content of the eluate increased, the retention time of the amino acids decreased (capacity factor) resulting in an increased separation factor.

Table 1 Optical Separation of Racemers

| | 18 ℃ | | | | 2℃ | | | |
|---|---|---|---|---|---|---|---|---|
| | $K_L'$ | $K_D'$ | α | Rs | $K_L'$ | $K_D'$ | α | Rs |
| Alanine | 0.31 | 0.51 | 1.64 | 0.97 | 0.48 | 0.98 | 2.04 | 2.44 |
| valine | 1.18 | 1.18 | 1.00 | --- | 1.35 | 1.49 | 1.10 | 0.45 |
| Norvaline | 1.38 | 1.91 | 1.39 | 2.15 | 1.73 | 2.90 | 1.67 | 3.85 |
| Leucine | 4.09 | 5.32 | 1.30 | 2.79 | 4.70 | 7.09 | 1.51 | 4.21 |
| Norleucine | 4.50 | 5.93 | 1.32 | 2.64 | 5.62 | 8.54 | 1.52 | 4.40 |
| a-Amino Butyrate | 0.60 | 0.76 | 1.27 | 0.71 | 0.64 | 1.01 | 1.57 | 1.79 |
| Isoleucine | 3.23 | 3.51 | 1.09 | 0.76 | 3.92 | 4.48 | 1.14 | 1.32 |
| Phenylalanine | 12.62 | 13.83 | 1.10 | 1.17 | 18.52 | 20.93 | 1.13 | 1.55 |
| DOPa | 5.52 | 6.12 | 1.11 | 1.15 | 12.76 | 14.53 | 1.14 | 1.53 |

| | | | | | | | | |
|---|---|---|---|---|---|---|---|---|
| Phenylglycine | 2.45 | 8.27 | 3.37 | 8.63 | 3.19 | 19.32 | 6.06 | 14.47 |
| Methionine | 2.29 | 3.72 | 1.63 | 3.99 | 3.33 | 7.08 | 2.12 | 6.99 |
| Threonine | 0.21 | 0.30 | 1.44 | 0.37 | 0.27 | 0.58 | 2.17 | 1.61 |
| Cysteine | 0.31 | 0.44 | 1.43 | 0.46 | 0.42 | 0.79 | 1.89 | 1.50 |
| Tyrosine | 9.42 | 10.24 | 1.09 | 1.04 | 20.74 | 22.97 | 1.11 | 1.22 |
| Asparginate | 0.23 | 0.40 | 1.76 | 0.54 | 0.43 | 0.80 | 1.84 | 1.68 |
| Glutamate | 0.43 | 0.83 | 1.91 | 1.69 | 0.75 | 3.25 | 4.33 | 7.92 |
| Glutamine | 0.23 | 0.64 | 2.75 | 1.97 | 0.40 | 1.75 | 4.37 | 4.98 |
| Lysine | 0.81 | 0.81 | 1.00 | --- | 2.00 | 2.36 | 1.18 | 0.97 |
| Arginine | 0.47 | 0.81 | 1.73 | 1.61 | 0.92 | 2.40 | 2.60 | 4.11 |
| Phenylethylamine | 19.70 | 17.37 | 1.13 | 0.77 | 22.85 | 27.66 | 1.21 | 1.67 |
| a-Amino- -Caprolactam | 1.31 | 1.86 | 1.42 | 2.13 | 1.81 | 3.15 | 1.74 | 3.88 |

**Table 2 Optical Separation of Amino Acids and Racemers**

| | R-1 | | | | R-2 | | | |
|---|---|---|---|---|---|---|---|---|
| | $k_L'$ | $k_n'$ | α | Rs | $k_L'$ | $k_n'$ | α | Rs |
| Alanine | 0.05 | 1.02 | 2.03 | 2.09 | 0.56 | 1.04 | 1.86 | 1.73 |
| valine | 0.98 | 1.22 | 1.25 | 0.86 | 0.88 | 1.02 | 1.16 | sh |
| Methionine | 2.26 | 5.90 | 2.61 | 4.68 | 2.16 | 4.61 | 2.14 | 3.03 |
| Phenylalanine | 8.73 | 13.22 | 1.51 | 2.33 | 7.59 | 9.49 | 1.25 | 1.11 |
| Phenylglycine | 2.81 | 17.54 | 6.25 | 7.39 | 2.70 | 12.76 | 4.73 | 6.56 |
| Threonine | 0.31 | 0.53 | 1.69 | 1.08 | 0.32 | 0.59 | 1.86 | 1.26 |
| Tyrosine | 6.15 | 9.39 | 1.53 | 2.38 | 6.29 | 8.13 | 1.29 | 1.03 |
| Glutamate | 0.59 | 2.34 | 3.95 | 5.05 | 0.66 | 2.38 | 3.63 | 3.87 |
| Arginine | 0.71 | 1.36 | 1.93 | 2.10 | 0.88 | 1.80 | 2.06 | 1.75 |

Table 3 Column Organic Solvent Resistance

| Eluate / Column | 0%MeOH | 10%MeOH | 20%MeOH | 30%MeOH | 40%MeOH |
|---|---|---|---|---|---|
| R-1 | -- | $3.5 \times 10^{-7}$ | $6.7 \times 10^{-7}$ | $9.6 \times 10^{-6}$ | $1.5 \times 10^{-4}$ |
| R-2 | -- | -- | -- | -- | -- |

**Table 4 Optical Separation of Amino Acids Using Methanol-Containing Eluate**

| | | 0%MeOH | | | 10%MeOH | | | 20%MeOH | | | 30MeOH | | | 40%MeOH | | |
|---|---|---|---|---|---|---|---|---|---|---|---|---|---|---|---|---|
| | | k' | α | Rs | k' | α | Rs | k' | α | Rs | k' | α | Rs | k' | α | Rs |
| Glutamate | L | 0.61 | 3.63 | 3.87 | 0.58 | 4.17 | 3.65 | 0.54 | 4.78 | 3.61 | 0.53 | 5.15 | 4.12 | 0.50 | 5.41 | 4.02 |
| | D | 2.38 | | | 2.42 | | | 2.60 | | | 2.74 | | | 2.73 | | |
| Arginine | L | 0.88 | 2.06 | 1.75 | 0.63 | 2.27 | 1.75 | 0.47 | 2.39 | 1.83 | 0.39 | 2.50 | 1.79 | 0.33 | 2.53 | 1.56 |
| | D | 1.80 | | | 1.43 | | | 1.13 | | | 0.97 | | | 0.83 | | |
| Methionine | L | 2.16 | 2.14 | 3.03 | 1.60 | 2.50 | 3.34 | 1.35 | 3.00 | 3.45 | 1.17 | 3.48 | 3.82 | 1.01 | 3.91 | 3.58 |
| | D | 4.61 | | | 4.01 | | | 4.06 | | | 4.08 | | | 3.96 | | |
| Phenylglycine | L | 2.70 | 4.73 | 6.56 | 2.00 | 5.69 | 6.11 | 1.78 | 6.71 | 5.82 | 1.39 | 7.88 | 5.56 | 1.14 | 8.64 | 5.05 |
| | D | 12.76 | | | 11.39 | | | 11.92 | | | 10.97 | | | 9.82 | | |
| Phenylalanine | L | 7.59 | 1.25 | 1.11 | 4.32 | 1.37 | 1.38 | 3.04 | 1.54 | 1.70 | 2.11 | 1.79 | 1.98 | 1.56 | 2.00 | 1.91 |
| | D | 9.49 | | | 5.90 | | | 4.68 | | | 3.77 | | | 3.12 | | |

Table 4

| | | | | | | | | | | | | | | | | | |
|---|---|---|---|---|---|---|---|---|---|---|---|---|---|---|---|---|---|
| Tyrosine | L | 6.29 | 1.29 | 1.03 | 3.17 | 1.49 | 1.57 | 2.23 | 1.75 | 1.92 | 1.50 | 2.05 | 2.27 | 1.12 | 2.28 | 2.19 |
| | D | 8.13 | | | 4.72 | | | 3.90 | | | 3.08 | | | 2.56 | | |
| Tryptophan | L | 47.70 | 1.15 | 0.73 | 21.45 | 1.27 | 1.24 | 12.52 | 1.44 | 1.58 | 7.26 | 1.66 | 1.80 | 4.55 | 1.89 | 1.68 |
| | D | 54.89 | | | 27.25 | | | 17.98 | | | 12.07 | | | 8.59 | | |

Embodiment 2

A: Synthesis of 6'6'-dioctadecyl-2,2'-dimethoxy-1,1'-binaphthyl

4 ml of bromine were dropped into a dichloromethane solution containing 10g of 2,2'-dimethoxy-1,1'-binaphthyl (S isomer) over a 20 minutes period in a 0 ° C nitrogen atmosphere, and stirred for 30 minutes. 60 ml of 20% $NaHSO_3$ were then added to decompose the unreacted bromine. 200ml of water were added to the reaction solution and after vigorous stirring, the solution was filtered with a glass filter, the residue then being washed with methylene chloride (1). After separating off the mother liquor, the organic phase was evaporated under reduced pressure. 20ml of chloroform were added to the residue, filtered, and washed with chloroform(2). The filtrate was evaporated and purified by column chromatography (silica gel, chloroform/hexane) (3). It could be seen from NMR and mass spectroscopy, that (1), (2) and (3) were all the target substances. The amount obtained was 12.8g and the yield was 83.6% (Compound III).

80ml of dry ether were added to 1.65g of magnesium and a solution in which 22g of stearyl bromide were dissolved in 60ml of ether in a nitrogen atmosphere was slowly dropped in. Following dropping, and refluxing while heating for 1 hour the solution was cooled after. A suspension of 4g of Compound 1 and 320mg of dichloro [1,3-bis(diphenylphosphine)propane]nickel in 150ml of ether was added and refluxed for 2 hours. After cooling, 50ml of water and 70 ml of 1N hydrochloric acid were added. After stirring for 1 hour, extract ion was carried out using 200ml of 0.5N hydrochloric acid and 400ml of chloroform. The organic layer was dried and evaporated under reduced pressure and purified with column chromatography (silica gel, chloroform/hexane) to obtain the pure reaction product. The amount obtained was 5.4mg and the yield was 78% ($[a]^D$ = +9.05(c=1.05 in THF) (Compound 2).

B: Synthesis of 6,6'-dioctadecyl-3,3'-diphenyl-2,2'-dihydroxy-1,1'-binaphthyl

3.1ml of tetramethylethylenediamine and 12ml of n-BuLi were added to 100ml of dry ether and stirred for 20 minutes in a nitrogen atmosphere at room temperature. 5.2g of Compound 2 were added to this followed by additional stirring for 20 minutes at room temperature so that compound 2 completely decomposed resulting in a wine red-colored homogeneous solution. This solution was cooled to -50°C followed by slowly dropping in a solution of 3ml of bromine diluted in 50ml of hexane. After stirring for 20 minutes, the temperature of the solution was gradually returned to room temperature. At room temperature, the unreacted bromine was dissolved with a saturated aqueous solution of sodium sulfite. The product was then extracted with chloroform and purified by column chromatography (silica gel, chloroform/hexane) to obtain 1.99g of pure 6,6'-dioctadecyl-3,3'-dibromo-2,2'-dimethoxy-1,1'-binaphthyl. (yield 32%) (Compound 3).

A suspension of 80mg of $Ni[PPh_3]_2Cl_2$ and 20ml of ether were added to 1.9g of Compound 3, and stirred for 15 minutes in a nitrogen atmosphere. 10mmol of phenylmagnesium bromide were added and refluxed for 4 hours. After cooling the solution was quenched with 20ml of 1N hydrochloric acid and extracted with chloroform. The organic layer was evaporated under reduced pressure, and purified by column chromatography (silica gel, hexane/benzene)to obtain 670mg of pure 6,6'-dioctadecyl-3,3'-diphenyl-2,2'-dimethoxy-1,1'-binaphthyl. (yield 36%) (Compound 4).

20ml of methylene chloride were added to 670mg of Compound 4. Following addition of 2ml of $BBr_3$ at 0°C in a nitrogen atmosphere, the solution was stirred for 6 hours at room temperature. After quenching the reaction solution with 30ml of water, the solution was extracted with chloroform and purified by column chromatography (silica gel, hexane/benzene) to obtain 560mg of pure 6,6'-dioctadecyl-3,3'-diphenyl-2,2'-dihydroxy-1,1'-binaphthyl with a yield of 86% (Compound 5).

C: Synthesis of 2,3:4,5-bis[1,2-(3-phenyl-6-octadecylnaphtho)]1,6,9,12,15,18-hexaeicosa-2,4-diene

520mg of Compound 5 were dissolved in 100ml of THF in a nitrogen atmosphere followed by the addition of 0.25g of KOH and 330mg of pentaethylene glycol ditosylate. After refluxing the mixture for 72 hours, the product was extracted with chloroform and purified with column chromatography (alumina, chloroform/hexane) to obtain 320mg of the pure target compound. The yield was 51%.

| | |
|---|---|
| Specific Rotation: | $[a]^D$ = -8.5, $[a]^{577}$ = 7.7, $[a]^{546}$ = -8.5, $[a]^{435}$ = -11.7 (c=1.01 in THF) |
| Mass Spectrum: | m/e 1144 ($M^+$) |
| 'H NMR (60MHz,$CDCl_3$): | = 0.86(t,$CH_3$,6H), 1.23(m,other $CH_2$,64H), 2.72(t,a-$CH_2$,4H) 3.27-(m,$OCH_2$,20H), 7.42(m,ArH,18H) |

Embodiment 3

A: Preparation of Optical Isomer Separation Column

104mg of 2,3:4,5-bis[1,2-(3-phenyl-6-octadecylnaphtho)] 1,6,9,12,15,18-hexaeicosa-2,4-diene were dissolved in a solvent mixture of 27ml of methanol and 10ml of THF. This solution was then circulated in a column (inner diameter: 4mm, length: 125mm) filled with commercially available octadecyl silica (ODS, particle diameter: 5u) using a high-pressure pump, and was allowed to circulate inside the column for 6 hours. Water was sequentially added to the circulating liquid and gradually the ratio of organic solvent was decreased As a result, the above substance was nearly completely adsorbed onto the ODS in the column.

B: Separation of Optical Isomers

An example of optical resolution of racemic amino acids with HPLC is indicated in Table 5. In this case, an aqueous solution of perchloric acid of pH 2.0 was used as the eluate, the column temperatures were 18°C and 2°C, 2μl of the sample of a concentration of 5mM were injected and the sample was detected by UV absorption at 200nm.

Table 5

| | 2℃ 0.5ml/min. | | | | 18℃ 0.5ml/min. | | | |
|---|---|---|---|---|---|---|---|---|
| | $k^1$ | $k^2$ | α | RS | $k^1$ | $k^2$ | α | RS |
| Alanine | 0.49 | 0.86 | 1.76 | 1.82 | 0.33 | 0.48 | 1.44 | 1.00 |
| Valine | 1.01 | 1.01 | 1.0 | --- | 0.82 | 0.82 | 1.0 | --- |
| Leucine | 3.14 | 4.45 | 1.42 | 1.76 | 2.45 | 3.08 | 1.26 | 1.20 |
| Isoleucine | 2.55 | 2.93 | 1.15 | 0.66 | 2.05 | 2.05 | 1.0 | --- |
| Phenylalanine | 11.69 | 13.20 | 1.13 | 1.40 | 6.78 | 6.78 | 1.0 | --- |
| DOPA | 8.92 | 10.16 | 1.14 | 0.79 | 3.67 | 4.01 | 1.09 | 1.03 |
| Methionine | 2.37 | 4.60 | 1.94 | 2.90 | 1.51 | 2.27 | 1.51 | 2.07 |
| Phenylglycine | 2.34 | 11.35 | 4.85 | 4.26 | 1.63 | 4.63 | 2.84 | 3.76 |
| Serine | 0.30 | 0.40 | 1.32 | 0.98 | 0.25 | 0.25 | 1.0 | --- |
| Threonine | 0.31 | 0.55 | 1.75 | 1.39 | 0.32 | 1.32 | 1.0 | --- |
| Tyrosine | 13.71 | 14.92 | 1.09 | 1.82 | 5.63 | 5.63 | 1.0 | --- |

Table 5

| | | | | | | | | |
|---|---|---|---|---|---|---|---|---|
| Asparagine | 0.30 | 0.40 | 1.32 | 1.20 | 0.25 | 0.25 | 1.0 | --- |
| Asparginate | 0.47 | 0.70 | 1.62 | 1.47 | 0.29 | 0.29 | 1.0 | --- |
| Glutamate | 0.69 | 2.47 | 3.60 | 3.75 | 0.41 | 0.96 | 2.33 | 2.17 |
| Ornithine | 0.85 | 1.23 | 1.44 | 1.06 | 0.49 | 0.49 | 1.0 | --- |
| Lysine | 1.60 | 1.79 | 1.12 | 0.93 | 0.67 | 0.67 | 1.0 | --- |
| Arginine | 0.71 | 0.71 | 2.42 | 2.32 | 0.42 | 0.67 | 1.58 | 1.07 |
| Histidine | 0.46 | 0.46 | 1.0 | --- | --- | --- | 1.0 | --- |
| Tryptophan | 95.99 | 95.55 | 1.0 | --- | 37.59 | 37.59 | 1.0 | --- |
| Glutamine | | | | | | | | |

## Claims

1. A crown ether compound represented by the general formula (I)

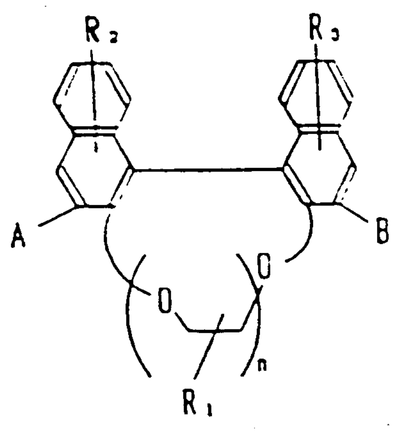

(I)

wherein A and B which may be the same or different represent a hydrogen atom, a straight chain or branched alkyl group having 1-6 carbon atoms an aryl group having 6-10 carbon atoms, or an aralkyl group having 7-9 carbon atoms;

$R_1$ represents a hydrogen atom, a straight chain or branched alkyl group having 1-20 carbon atoms and may be bonded to any carbon atom of the cyclic polyether the carbon number of which is from 1-12;

n represents an integer from 3-10;

$R_2$ and $R_3$ which may be the same or different represent a straight chain or branched alkyl group having 1-30 carbon atoms, an aryl group having 6-18 carbon atoms, or an aralkyl group having 7-30 carbon atoms, the location of $R_2$ and $R_3$ being anywhere else than the A and B substitution positions on the condensed ring, and although one of each of $R_2$ and $R_3$ is satisfactory, a maximum of 5 groups may be substituted.

**2.** The crown ether compound of claim 1 represented by the general formula (II)

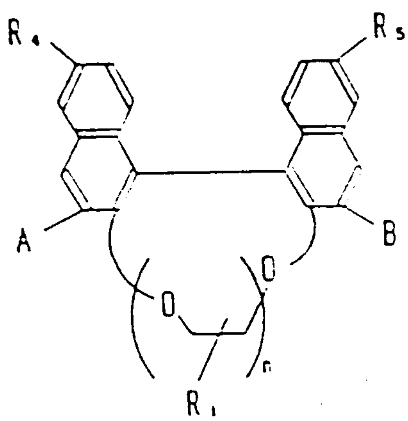

(II)

wherein A, B and $R_1$ are as defined in claim 1 and $R_4$ and $R_5$ each represents a straight-chain or branched alkyl group having 4-30 carbon atoms.

**3.** A separating agent comprising an optically active crown ether compound of claim 1 or 2 being adsorbed on a carrier.

**Patentansprüche**

**1.** Kronenetherverbindung, dargestellt durch die allgemeine Formel (I)

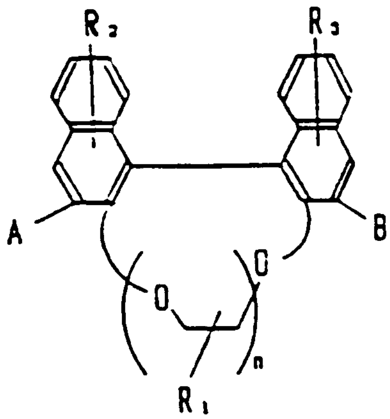

(I)

worin A und B, die gleich oder verschieden sein können, ein Wasserstoffatom, eine geradkettige oder verzweigte Alkylgruppe mit 1 bis 6 Kohlenstoffatomen, eine Arylgruppe mit 6 bis 10 Kohlenstoffatomen oder eine Aralkylgruppe mit 7 bis 9 Kohlenstoffatomen darstellen;

$R_1$ ein Wasserstoffatom, eine geradkettige oder verzweigte Alkylgruppe mit 1 bis 20 Kohlenstoffatomen darstellt und an irgendein Kohlenstoffatom des cyclischen Polyethers mit einer Kohlenstoffzahl von 1 bis 12 gebunden sein kann;

n eine ganze Zahl von 3 bis 10 ist;

$R_2$ und $R_3$, die gleich oder verschieden sein können, eine geradkettige oder verzweigte Alkylgruppe mit 1 bis 30 Kohlenstoffatomen, eine Arylgruppe mit 6 bis 18 Kohlenstoffatomen oder eine Aralkylgruppe mit 7 bis 30 Kohlenstoffatomen darstellen und die Position von $R_2$ und $R_3$ irgendwo anders als die Substitutionspositionen von A und B an dem kondensierten Ring sind, und obwohl jeweils ein $R_2$ und $R_3$ ausreichend sind, kann ein Maximum von 5 Gruppen substituiert sein.

**2.** Kronenetherverbindung nach Anspruch 1, dargestellt durch die allgemeine Formel (II)

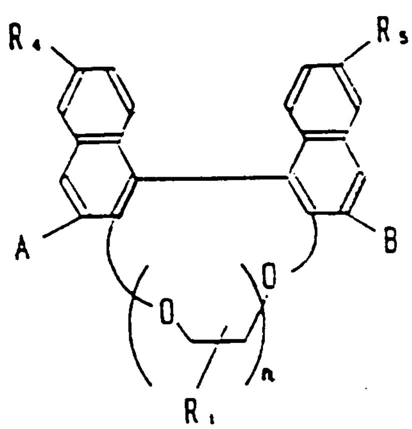

(II)

worin A, B und $R_1$ wie in Anspruch 1 definiert sind, und $R_4$ und $R_5$ jeweils eine geradkettige oder versweigte Alkylgruppe mit 4 bis 30 Kohlenstoffatomen darstellen.

**3.** Trennmittel, umfassend eine optisch aktive Kronenetherverbindung nach Anspruch 1 oder 2, die an einen Träger adsorbiert ist.

**Revendications**

**1.** Ether-couronne représenté par la formule générale I :

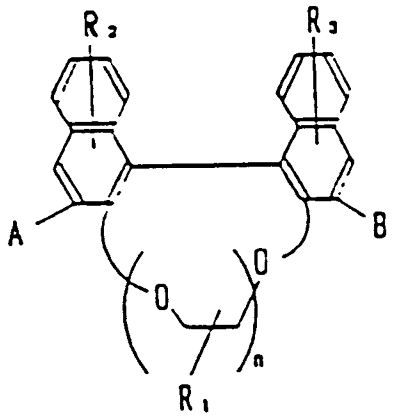

I

dans laquelle A et B, qui peuvent être identiques ou différents, représentent un atome d'hydrogène, un groupe alkyle en $C_1$-$C_6$ à chaîne linéaire ou ramifiée, un groupe aryle en $C_6$-$C_{10}$, ou un groupe aralkyle en $C_7$-$C_9$ ;

$R_1$ représente un atome d'hydrogène, un groupe alkyle en $C_1$-$C_{20}$ à chaîne linéaire ou ramifiée, et peut être lié à un atome de carbone quelconque du polyéther cyclique, dont le nombre d'atomes de carbone est compris entre 1 et 12 ;

n représente un nombre entier de 3 à 10 ;

$R_2$ et $R_3$, qui peuvent être identiques ou différents, représentent un groupe alkyle en $C_1$-$C_{30}$ à chaîne linéaire ou ramifiée, un groupe aryle en $C_6$-$C_{18}$, ou un groupe aralkyle en $C_7$-$C_{30}$, $R_2$ et $R_3$ pouvant être localisés en toutes autres positions que celles occupées par les substituants A et B sur le cycle condensé, et bien que un de chacun des groupes $R_2$ et $R_3$ soit suffisant, il peut exister jusqu'à 5 groupes substituants.

**2.** Ether-couronne selon la revendication 1, représenté par la formule générale II :

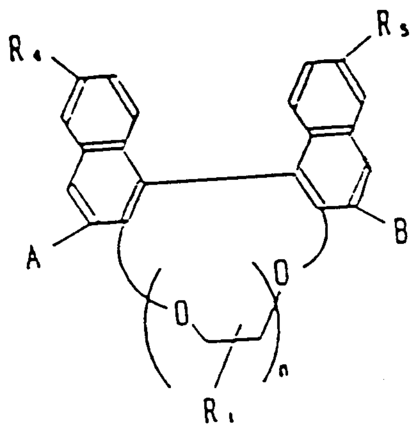

II

dans laquelle A, B et $R_1$ sont tels que définis à la revendication 1, et $R_4$ et $R_5$ représentent chacun un groupe alkyle en $C_4$-$C_{30}$ à chaîne linéaire ou ramifiée.

**3.** Agent de séparation comprenant un éther-couronne optiquement actif selon la revendication 1 ou 2, adsorbé sur un support.